# EUROPEAN PATENT SPECIFICATION

(11) **EP 2 212 391 B1**
(45) Date of publication and mention of the grant of the patent: **02.07.2014**
(21) Application number: 08845192.7
(22) Date of filing: 28.10.2008
(51) Int. Cl.: C09D 167/04, A61L 31/10

(54) **POLYMER BLENDS FOR DRUG DELIVERY STENT MATRIX WITH IMPROVED THERMAL STABILITY**
POLYMERMISCHUNGEN FÜR WIRKSTOFFABGABESTENTMATRIX MIT VERBESSERTER THERMOSTABILITÄT
MÉLANGES DE POLYMÈRES POUR UNE MATRICE DE STENT POUR L'ADMINISTRATION DE MÉDICAMENTS QUI PRÉSENTE UNE STABILITÉ THERMIQUE AMÉLIORÉE

(30) Priority: 31.10.2007 US 982168
(43) Date of publication of application: 04.08.2010
(73) Proprietor: Abbott Cardiovascular Systems Inc., Santa Clara, CA 95054-2807 (US)
(72) Inventor: LIM, Florencia, Union City CA 94587 (US); MASLANKA, Bozena, Zofia, Aptos CA 95003 (US); TANG, Yiwen, San Jose CA 95117 (US); TROLLSAS, Mikael, O., San Jose CA 95124 (US)
(74) Representative: ZBM Patents - Zea, Barlocci & Markvardsen
(86) International application number: PCT/US2008/081469
(87) International publication number: WO 2009/058786

(56) References cited:
- EP-A- 1 577 346
- WO-A-01/94439
- US-A1- 2005 233 062
- US-A1- 2006 093 771

## Description

### BACKGROUND

### Field of the Invention

This invention is generally related to a polymeric blend composition used for coating a medical device that exhibits improved thermal stability. The invention also encompasses implantable medical devices coated with the aforementioned coating.

### Description of the State of the Art

Percutaneous coronary intervention (PCI) is a procedure for treating heart disease. A catheter assembly having a balloon portion is introduced percutaneously into the cardiovascular system of a patient via the brachial or femoral artery. The catheter assembly is advanced through the coronary vasculature until the balloon portion is positioned across an occlusive lesion, which is limiting the blood flow to the coronary muscles. Once in position across the lesion, the balloon is inflated to a predetermined size to radially compress the atherosclerotic plaque of the lesion to remodel the lumen wall. The balloon is then deflated to a smaller profile to allow the catheter to be withdrawn from the patient's vasculature.

Problems associated with the above procedure include formation of intimal flaps or torn arterial linings which can collapse and occlude the blood conduit after the balloon is deflated. Moreover, thrombosis and restenosis of the artery may develop over several months after the procedure, which may require another angioplasty procedure or a surgical by-pass operation. To reduce the partial or total occlusion of the artery by the collapse of the arterial lining and to reduce the chance of thrombosis or restenosis, a stent is implanted in the artery to keep the artery open.

Drug delivery stents have reduced the incidence of in-stent restenosis (ISR) after PCI (see, e.g., Serruys, P.W., et al., J. Am. Coll. Cardiol. 39:393-399 (2002)), which has been a concern for interventional cardiology since its beginning. However, given the large volume of coronary interventions and their expanding use ISR still poses a significant problem for the medical community. Additionally, the use of drugs and polymer eluting drug matrices have raised a safety concern for drug eluting stents. The pathophysiological mechanism of ISR involves interactions between the cellular and acellular elements of the vessel wall and the blood. Damage to the endothelium during PCI constitutes a major factor for the development of ISR and is equally important considered a safety concern due to delayed healing after PCI (see, e.g., Kipshidze, N., et al., J. Am. Coll. Cardiol. 44:733-739 (2004)).

US 2005/0233 062 A1 discloses a method of manufacturing an implantable medical device including a coating comprising blends of biodegradable polymers.

The embodiments of the present invention address these concerns as well as others that are apparent to one having ordinary skill in the art.

### SUMMARY OF THE INVENTION

The present invention relates to a coating that comprises a polymer blend composition, the polymer blend composition comprising: a semi-crystalline polymer with a weight-average-molecular-weight from 75,000 to 300,000; an amorphous, or substantially amorphous, polymer with a weight-average-molecular weight from 75,000 to 300,000 and wherein the amorphous, or substantially amorphous, polymer is selected from the group consisting of poly(D,L-lactic acid-co-glycolic acid) (PLGA), polyethylene glycol-blockpoly(D,L-lactic acid) (PEG-PLA), polyethylene glycol-block poly(D,L-lactic acid-co-glycolic acid) (PEG-PLGA), poly(lactide-glycolide-caprolactone)terpolymers, and combinations thereof; wherein the semi-crystalline polymer is between 2% and 75% by weight of the sum of the semi-crystalline and the amorphous, or substantially amorphous, polymer; wherein the effective glass transition temperature of the coating is - 60 °C or higher; the melting transition of a crystalline polymer region, or at least one melting transition of a polymer crystalline region if there are more than one polymer melt transitions, is 70 °C or higher; the coating comprises 0.5% to 50% by weight polymer crystallinity; and the effective glass transition temperature is the measured glass transition temperature before exposure to ethylene oxide sterilization and, if more than one glass transition temperature is observed for the blend, then the effective glass transition temperature is the lower of the two or more distinctly observable glass transition temperature values.

It also relates to an implantable medical device comprising a coating, the coating comprising: a polymer blend composition, the polymer blend composition comprising: a semi-crystalline polymer with a weight-average-molecular-weight from 75,000 to 300,000; an amorphous, or substantially amorphous, polymer with a weight-average-molecular weight from 75,000 to 300,000; wherein the semi-crystalline polymer is between 2% and 75% by weight of the sum of the semi-crystalline and the amorphous, or substantially amorphous, polymer; the semi-crystalline polymer is selected from the group consisting of poly(D-lactic acid) (PDLA), poly(L-lactic acid) (PLLA), poly(L-lactic acid-co-glycolic acid) (PLLGA), poly(L-lactic acid-co-glycolide-co- caprolactone) (PLLA-GA-CL), and combinations thereof, and the amorphous, or substantially amorphous polymer is selected from the group consisting of poly(D,L-lactide-glycolide), poly(lactide-glycolide-caprolactone)terpolymers, and combinations thereof, wherein the effective glass transition of the coating is - 60 °C. or higher; the coating comprises 0.5% to 50% by weight polymer crystallinity; the coating has a water content of 10% or less after sterilization; and the effective glass transition temperature is the measured glass transition temperature before exposure to ethylene oxide sterilization and, if more than one glass transition temperature is observed for the blend, then the effective glass transition temperature is the lower of the two or more distinctly observable glass transition temperature values.

In some embodiments, the aforementioned coating has an effective glass transition of the coating is about - 60 °C or higher (it may have also have another glass transition) includes about 0.5% to about 50% by weight polymer crystallinity, and a melting temperature of the polymer crystalline regions of about 70 °C or higher.

In other embodiments, the aforementioned coating has an effective glass transition of the coating is about - 60 °C or higher (it may also have another glass transition), the coating has about 0.5% to about 50% by weight polymer crystallinity, and the coating has a water content of about 10 % or less after being subjected to sterilization, and the semi-crystalline polymer is selected from the group consisting of PDLA, PLLA, PLLGA, PLLA-GA-CL, and combinations thereof, and the amorphous, or substantially amorphous polymer is selected from the group consisting of poly(D,L-lactide-gylcolide), a copolymer comprising the structures originating from D,L-lactide, glycolide, and caprolactone monomers (to be referred to as poly(lactide-glycolide-caprolactone) terpolymers), amorphous poly (L-Lactide-glycolide-caprolactone) terpolymers, and combinations thereof. In still other embodiments, the aforementioned coating has an effective glass transition of the coating is about - 60 °C or higher, the coating has about 0.5% to about 50% by weight polymer crystallinity, and the coating is biodegradable and the time at which the coating has substantially, or completely, degraded is between about 1 month to about 12 months, and in addition, the semi-crystalline polymer selected from the group consisting of PDLA, PLLA, PLLGA, PLLA-GA-CL, and combinations thereof, and the amorphous, or substantially amorphous polymer is selected from the group consisting of poly(D,L-lactide-glycolide), a copolymer comprising the structures originating from D,L-lactide, glycolide, and caprolactone monomers, and amorphous poly (L-Lactide-glycolide-caprolactone), and combinations thereof.

Still other embodiments of the present invention include a coating that includes a polymer blend composition, the polymer blend composition, which includes a semi-crystalline polymer with a weight-average-molecular-weight from about 75,000 to about 300,000, and an amorphous, or substantially amorphous, polymer with a weight-average-molecular weight from about 75,000 to about 300,000, wherein the semi-crystalline polymer is between about 2% and about 75% by weight of the sum of the semi-crystalline and the amorphous, or substantially amorphous, polymer, the semi-crystalline polymer is selected from the group consisting of PDLA, PLLA, PLLGA, PLLA-GA-CL, and combinations thereof, and the amorphous, or substantially amorphous polymer, is selected from the group consisting of poly(D,L-lactide-glycolide) terpolymers, amorphous poly(lactide-glycolide-caprolactone) terpolymers, and combinations thereof. The aforementioned coating further has the properties that the coating includes about 0.5% to about 50% by weight polymer crystallinity, the coating is biodegradable and the time at which the coating has substantially, or completely, degraded is between about 1 month to about 18 months, and the coating has a dynamic shear storage modulus that is greater than the dynamic shear loss modulus, where both are measured at the temperature of and under the conditions of ethylene oxide sterilization, and measured in the linear viscoelastic range at 1 radian/second, and the dynamic shear loss modulus is about 2 x 10⁴ Pa or less.

Various embodiments of the present invention also include an implantable medical device coated with the any one or more of the aforementioned coatings.

### BRIEF DESCRIPTION OF THE DRAWINGS

FIG. 1 illustrates an exemplary embodiment of a stent.
FIGs. 2A - 2D illustrate scanning electron microscope (SEM) images of a stent having a coating made from poly(D,L-lactide-glycolide) 75/25 after a simulated use test, and which was sterilized with ethylene oxide.
FIG. 3 is an exemplary embodiment of a coating construct of the present invention.
FIGs. 4A - 4D illustrate scanning electron microscope (SEM) images of a stent having coating which is an exemplary embodiment of a coating of the present invention.
FIGs. 5A - 5C illustrate scanning electron microscope (SEM) images of a stent having coating which is an exemplary embodiment of a coating of the present invention.

### DETAILED DESCRIPTION

The present invention provides a coating including a polymer blend composition, and a medical device comprising the aforementioned coating. The polymer blend composition comprises one or more semi-crystalline polymers and one or more amorphous, or substantially amorphous, polymers. The coating utilizing the blend exhibits improved thermal stability and/or stability when exposed to elevated temperatures and ethylene oxide, and has an effective T_{g} that is about -60 °C or higher (although it may also have other glass transitions), and a weight % polymer crystallinity of about 0.5% or more.

The polymer blend composition includes one or more semi-crystalline polymers and one or more amorphous polymers, which are described below. In some embodiments, the polymer blend improves the thermal stability of the coating because high temperature, and/or high temperature in combination with the diffusion of ethylene oxide and/or water into the polymeric coating, do not significantly affect the mechanical stability of the polymer blend. Therefore, the effect of temperature and exposure to plasticizers such as ethylene oxide and/or water on the coatings' mechanical integrity is lower using this polymer coating. In some embodiments, the impact of ethylene oxide sterilization, that is the impact of absorbed ethylene oxide and/or water, as well as the elevated temperature, do impact the mechanical properties, but the mechanical properties, such as a the tensile or compressive modulus, the creep compliance, and the dynamic shear and loss moduli, do not decrease below a level that maintains coating mechanical integrity. In addition, the addition of the crystallinity in the semi-crystalline polymer is expected to increase the toughness of the polymer.

Water and/or ethylene oxide may plasticize the polymer, thus reducing the T_{g} of a polymer. In some embodiments, the coating including the polymer blend composition, or the polymer blend itself, has an effective T_{g} is about - 60 °C or higher. In other embodiments, the coating including the polymer blend composition, or the polymer blend composition itself, may be chosen to ensure that the effect of water uptake and/or the effect of ethylene oxide uptake on mechanical properties are limited. This assures that the effective T_{g}, which is reduced under the conditions of sterilization, has less influence on the mechanical integrity of the polymer coating.

In some embodiments, the weight % polymer crystallinity in the coating may be about 0.1% to about 50%. In still other embodiments, the weight % polymer crystallinity may be expressed as a weight % of the total polymer in the coating, rather than the weight % of the coating. Thus in some embodiments, about 0.1 % to about 50% by weight of the polymer in the coating may be crystalline polymer domains.

The coating described herein can be degradable or durable. In some embodiments, the coating may degrade within (or in other words, the time period at which the coating has degraded, or substantially degraded) about 1 month, 2 months, 3 months, 4 months, 6 months, 12 months, 18 months, or 24 months after implantation of a medical device including the coating. In some embodiments, the coating may completely degrade, or absorb within 24 months after implantation of a medical device including the coating. In some embodiments utilizing a bioabsorbable polymer or other bioabsorbable material, very negligible traces or residue may be left behind.

The coating described herein can be formed on an implantable device such as a stent, which can be implanted in a patient to treat, prevent, mitigate, or reduce a vascular medical condition, or to provide a pro-healing effect. In some embodiments, the coating can include one or more bioactive agents, e.g., drug(s).

### Definitions

Wherever applicable, the definitions to some terms used throughout the description of the present invention as provided below shall apply. Unless defined otherwise, all technical and scientific terms used herein have the same meaning as commonly understood by one of ordinary skill in the art to which the invention belongs.

A "polymer" is broadly defined as "a molecule made up by the repetition of some simpler unit, the mer, or the monomer" (Ferdinand Rodriguez, Principles of Polymers Systems, Taylor and Francis, Bristol PA 1996). "Polymer" or "polymeric" refer to compounds that are the product of a polymerization reaction. The term polymer, or polymeric are inclusive of homopolymers (i.e., polymers obtained by polymerizing one type of monomer), copolymers (i.e., polymers obtained by polymerizing two or more different types of monomers), terpolymers (polymers obtained by polymerizing three different types of monomers), etc., including random, alternating, block, graft, dendritic, and any other variations thereof, including cross-linked polymers, and interpenetrating networks.

In some embodiments, the term "domain" may be referred to as "phase." Therefore, the term "crystalline domain" can be referred to as "crystalline phase." Similarly, the term "amorphous domain" may be referred to as "amorphous phase."

The "glass transition temperature," T_{g}, is the temperature at which the amorphous domains of a polymer change from a brittle, vitreous state to a solid deformable state (or rubbery state) at atmospheric pressure. In other words, the T_{g} corresponds to the temperature where the onset of segmental motion in the chains of the polymer occurs. The measured T_{g} of a given polymer can be dependent on the heating rate and can be influenced by the thermal history, and potentially pressure history, of the polymer, as well as potentially the pressure at which the measurement is made. T_{g} is also affected by other compounds mixed with the polymer, whether it is a filler, solvent, excipient, drug, plasticizer or compounds diffusing into the polymer during processing, such as water and/or ethylene oxide during sterilization.

As used herein, "effective T_{g}" for a coating including one of the polymer blend compositions of the present invention, or the polymer blend itself, will refer to the measured T_{g} before exposure to ethylene oxide sterilization, unless specified otherwise. If more than one T_{g} is observed for the blend, then the "effective T_{g}" refers to the lower of the two or more distinctly observable T_{g} values.

The "melting temperature", Tₘ, of a polymer is the highest temperature at which a crystal lattice in the polymer is stable.

The term "polymer crystallinity", as used herein, will refer to the % crystallinity in a given composition that is due to polymer components, as opposed to other materials (e.g. drug).

The terms "biologically degradable" (or "biodegradable"), "biologically erodable" (or "bioerodable"), "biologically absorbable" (or "bioabsorbable"), and "biologically resorbable" (or "bioresorbable"), as well as degraded, eroded, absorbed, and dissolved, in reference to polymers, coatings, or other materials referenced herein, are used interchangeably, and refer to polymers, coatings, and materials that are capable of being completely or substantially completely, degraded, dissolved, and/or eroded over time when exposed to physiological conditions, and can be gradually resorbed, absorbed and/or eliminated by the body, or that can be degraded into fragments that can pass through the kidney membrane of an animal (e.g., a human), e.g., fragments having a molecular weight of about 40,000 Daltons (40 kDa) or less. The process of breaking down and eventual absorption and elimination of the polymer, coating, or other material can be caused by, e.g., hydrolysis, metabolic processes, oxidation, enzymatic processes, bulk or surface erosion, and the like. Conversely, a "biostable" polymer, coating, or material, refers to a polymer, coating or material that is not biodegradable.

As used herein, an "implantable device" may be any suitable substrate that can be implanted in a human or non-human animal. Examples of implantable devices include, but are not limited to, self-expandable stents, balloon-expandable stents, coronary stents, peripheral stents, stent-grafts, catheters, other expandable tubular devices for various bodily lumen or orifices, grafts, vascular grafts, arterio-venous grafts, by-pass grafts, pacemakers and defibrillators, leads and electrodes for the preceding, artificial heart valves, anastomotic clips, arterial closure devices, patent foramen ovale closure devices, cerebrospinal fluid shunts, and particles (e.g., drug-eluting particles, microparticles and nanoparticles).

"Drug" or "active agent" or "bioactive agent" or "therapeutic agent," all of which will be used interchangeably, refers to any substance that, when administered in a therapeutically effective amount to an individual (any animal, including a human) suffering from a disease or condition, or seeking medical treatment: (1) cures the disease or condition; (2) slows the progress of the disease or condition; (3) causes the disease or condition to retrogress; (4) alleviates one or more symptoms of the disease or condition; or (5) provides some other beneficial effect on the health and well-being of the individual. A drug also includes any substance that when administered to a individual, known or suspected of being particularly susceptible to a disease, in a prophylactically effective amount, has a prophylactic beneficial effect on the health and well-being of the patient, which includes but is not limited to: (1) preventing or delaying on-set of the disease or condition in the first place; (2) maintaining a disease or condition at a retrogressed level once such level has been achieved by a therapeutically effective amount of a substance, which may be the same as or different from the substance used in a prophylactically effective amount; or (3) preventing or delaying recurrence of the disease or condition after a course of treatment with a therapeutically effective amount of a substance, which may be the same as or different from the substance used in a prophylactically effective amount, has concluded. The term drug, as used herein in this specification including the appended claims, also encompasses agents useful has diagnostic agents. The term "drug" also encompasses pharmaceutically acceptable, pharmacologically active derivatives of those drugs specifically mentioned herein, including, but not limited to, salts, esters, amides, prodrugs, active metabolites, analogs, and the like. As used herein, the term "prodrug" refers to an agent rendered less active by a chemical or biological moiety, which metabolizes into or undergoes in vivo hydrolysis to form a drug or an active ingredient thereof.

As used herein, a material that is described as a layer or a film (e.g., a coating) "disposed over" an indicated substrate (e.g., an implantable device) refers to, e.g., a coating of the material deposited directly or indirectly over at least a portion of the surface of the substrate. Direct depositing means that the coating is applied directly to the exposed surface of the substrate. Indirect depositing means that the coating is applied to an intervening layer that has been deposited directly or indirectly over the substrate. A coating is supported by a surface of the device, whether the coating is deposited directly, or indirectly, onto the surface of the device.

"Sterilize" or "sterilization" refers to the process by which the bioburden of an item is reduced to a particular sterility assurance level (SAL), where the SAL is the probability of a viable microorganism being present on a product unit after the product has undergone sterilization procedure. The SAL level required will depend upon the use of the article.

"Delivery" and "deployment." With respect to an implantable medical device such as a stent, "delivery" refers to introducing and transporting the stent through a bodily lumen to a region, such as a lesion, in a vessel that requires treatment. Delivery of a stent is typically accomplished by crimping the stent onto a catheter, or a catheter balloon, inserting the end of the catheter through the skin into a bodily lumen, advancing the catheter in the bodily lumen to a desired treatment location where it is deployed. "Deployment" corresponds to the expanding of the stent within the lumen at the treatment region, typically by inflating the catheter balloon.

As used herein, the terms poly(D,L-lactide) (PLA), poly(L-lactide) (PLLA), poly(D,L-lactide-co-glycolide) (PLGA), and poly(L-lactide-co-glycolide) (PLLGA) are used interchangeably with the terms poly(D,L-lactic acid) (PLA), poly(L-lactic acid) (PLLA), poly(D,L-lactic acid-co-glycolic acid) (PLGA), and poly(L-lactic acid-co-glycolic acid) (PLLGA), respectively. Also the use of "co" is optional, and thus poly(L-lactide-co-glycolide) will be used interchangeably with poly(L-lactide-glycolide), etc.

In the discussion that follows, to avoid the stilted language required to consistently indicate that the plural of various aspects of this invention is included with the singular, any reference to the singular implies the plural and vice-versa, unless expressly stated to be otherwise; for example, "a bioactive agent" or "the bioactive agent" will refer to a single bioactive agent or to a plurality of bioactive agents; "a polymer" or "the polymer" will refer to a single polymer or a plurality of polymers; a "coating" or "the coating" will refer to a. single coating or a plurality of coatings, etc.

### Polymeric Blend Compositions

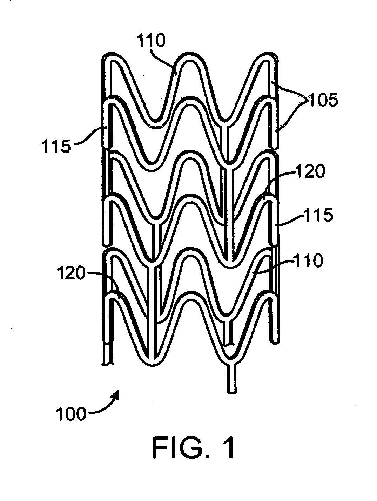

The present invention provides a polymeric blend composition used for coating a medical device, particularly an implantable medical device such as a stent. An example of a stent 100 is depicted in FIG. 1. In some embodiments, a stent may include a pattern or network of interconnecting structural elements or struts 105. Struts 105 of stent 100 include luminal faces or surfaces 110, abluminal faces or surfaces 115, and side-wall faces or surfaces 120. The embodiments disclosed herein are not limited to stents, or to the particular stent pattern, illustrated in FIG. 1. The embodiments are easily applicable to other patterns and other devices.

As stents are implanted in the body, stents must be sterilized. A number of techniques can be used to sterilize medical devices. Such processes are well known in the art. For medical devices in general, a number of sterilization methods can be used such as autoclaving, treatment with ethylene oxide, and irradiation, including, but not limited to, both gamma irradiation and electron beam (e-beam) irradiation. Most, if not all, of these processes can involve an elevated temperature.

For implantable medical devices, such as stents, ethylene oxide sterilization is often used. Ethylene oxide sterilization is performed by spraying or immersing the device in liquid ethylene oxide, or exposing the device to gaseous ethylene oxide to obtain a desired SAL. However, cycle times are long due to the need to assure that the ethylene oxide reaches all the areas requiring sterilization, particularly for complex devices, and to wait for the dissipation of the ethylene oxide from the items sterilized. To speed up this process, elevated temperatures may be used, but the elevated temperatures may have a negative impact on the product.

For example, ethylene oxide sterilization of a coated stent generally involves heating above 50°C at humidity levels reaching up to 100% for periods of a few hours up to 24 hours. A typical ethylene oxide cycle would have the temperature in the enclosed chamber to reach as high as above 50°C within the first 3-4 hours then and fluctuate between 40°C to 50°C for 17-18 hours while the humidity would reach the peak at 100% and remain above 80% during the fluctuation time of the cycle. The time and temperature of exposure varies with the type of device sterilized, and the desired SAL.

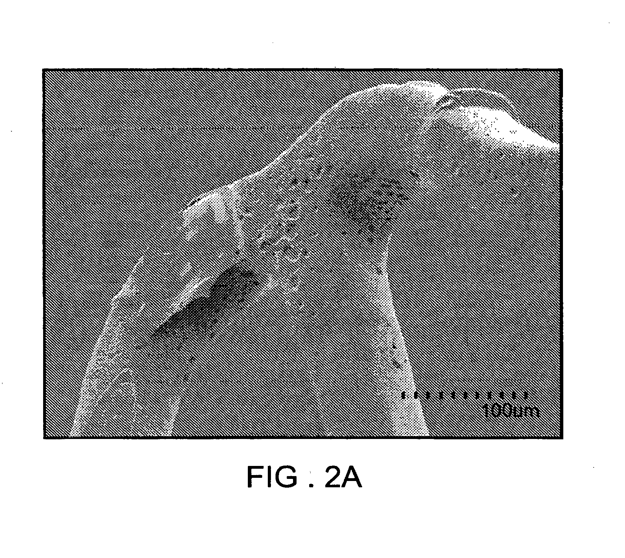

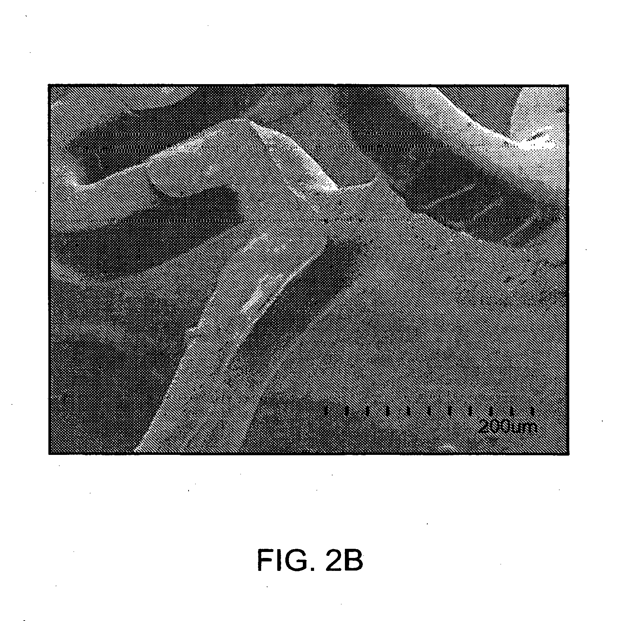

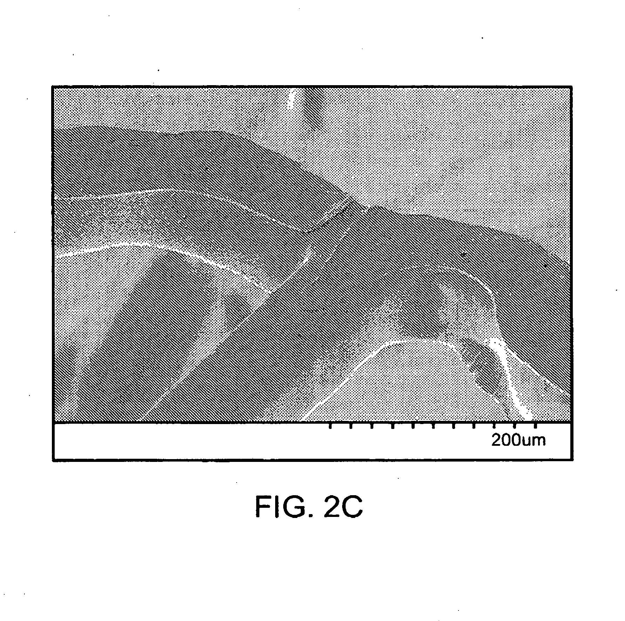

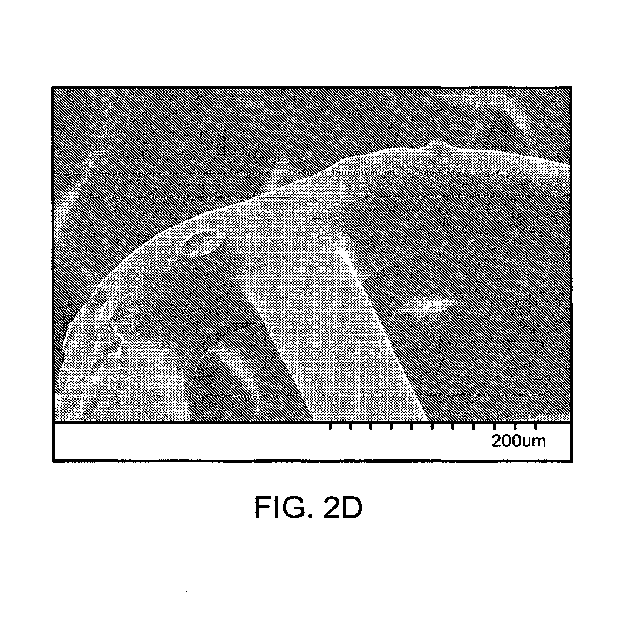

Any coating applied onto a stent must be able to function after being subjected to the stresses of ethylene oxide sterilization, and the mechanical stress and elevated temperature (around 70°C for a limited time) of crimping. FIGs 2A - 2D illustrate the impact of ethylene oxide sterilization on a coating of PLGA 72/25, poly(D,L-lactic acid-co-glycolic acid) (PLGA) with a molar ratio of lactide to glycolic acid of 75 to 25, over a primer coating of the same composition. As shown in the SEM images, the coating has been deformed. It is believed that the deformation is due to ethylene oxide and/or water uptake, which lowers the T_{g}, and allows the polymer to flow since there is no crystallinity to provide mechanical integrity. It is believed that the water uptake and/or uptake of ethylene oxide lowers the T_{g} such that it is below the sterilization temperature.

Subjecting the stent to sterilization by exposure to ethylene oxide often leads to absorption, or uptake, of ethylene oxide and/or water by the coating. For many polymeric materials, small molecules such was ethylene oxide and/or water may act as plasticizers, lowering . the T_{g} of the polymer. Furthermore, the absorption and subsequent desorption of water may deform the coating due to expansion and contraction of the coating.

Plasticization refers to the addition of a second, lower T_{g} and generally lower molecular weight material, to a polymer. The effect is to lower the T_{g} of the blend, and generally, also to transform a hard, brittle material to a soft, rubber-like material. According to the free volume model, the plasticizer, that is the second lower T_{g} and generally lower molecular weight material, added to the polymer, has a higher free volume. The addition of a higher free volume material to the polymer increases the "free volume" of the blend, and allows for greater polymer chain mobility, thus lowering the T_{g}. An alternative view, based on a lattice model similar to that used by Flory and Huggins, is that the true thermodynamic T_{g} is the point of zero configurational entropy. Thus, in this model, the lower T_{g} resulting from the addition of a second smaller molecule is due to the larger number of potential configurations of the polymer chains with the presence of the smaller molecule when compared to the number of potential configurations with only the long chain polymer molecules. Thus, regardless of the theoretical explanation for plasticization, the uptake of either water or ethylene oxide molecules or both, which are smaller than the polymer, would tend to allow for greater polymer chain mobility, and as a result, a lower T_{g}.

Thus, in some embodiments the water uptake, measured as weight percent water in the coating during the ethylene oxide sterilization process, may be limited to not more than 15%, In other embodiments, the weight % water in the coating, which includes the polymer blend composition, may be from about 0.1 to 15%, about 0.1 to about 10 %, from about 0.1 to about 7 %, or from about 0.1 % to about 6 %. In some embodiments, the increase in water content occurring during ethylene oxide sterilization, may be about 10 % or less, about 5 % or less, or about 0.5 % or less. In some embodiments, the water content (weight %) of the coating after ethylene oxide sterilization may be up to about 15%, up to about 10 %, up to about 7 %, or up to about 6%.

Water uptake and/or ethylene oxide uptake influence the T_{g} of the coating or the polymer blend. It is also known that crystalline regions in a polymer act as temperature dependent physical cross-links. Cross-linking of a polymer increases the glass transition temperature, increases the modulus and strength, and reduces the extent of creep, or viscous flow. Thus, in some embodiments, the increase in T_{g} due to the inclusion of crystalline domains, which act as physical cross-links, and/or the reduction in water uptake and/or reduction in uptake of ethylene oxide, may result in the coating including the polymer blend composition, with an effective T_{g} that is about 25 °C or higher, about 30 °C or higher, about 35°C or higher, about 40 °C or higher, about 45 °C or higher, about 50 °C or higher or about 55 °C or higher. In other embodiments, the polymer blend composition used in the coating may be chosen to ensure that the water uptake and/or the uptake of ethylene oxide are limited, and the effective T_{g} which may optionally also be impacted by the crystalline domains acting as physical cross-links, measured under the conditions of sterilization (with same level of plasticization due to both water and/or ethylene oxide), remains above the highest temperature encountered in sterilization, or remains above the temperature encountered in sterilization for about 80%, about 70%, or about 60%, or about 50%, of the time during the sterilization.

In some embodiments, the polymer blend composition used in the coating limits the water uptake and/or the uptake of ethylene oxide, to an amount such that the effective T_{g} in the amorphous regions, measured with the plasticization equivalent to that encountered in ethylene oxide sterilization, is not decreased by more than 30 °C. In other embodiments, the decrease in effective T_{g} occurring during the ethylene oxide sterilization may be about 15 °C, about 10 °C, about 5 °C, or about 20 °C. As outlined above, the effective preferred to here is defined as the T_{g} in the amorphous regions, or the lowest distinct T_{g} if more than one distinct T_{g} is present.

In other embodiments, the decrease in effective T_{g} may be about greater than 30 °C, and/or the effective T_{g} may be below the highest temperature encountered in either the ethylene oxide sterilization or the crimping operation, but the weight percent of the polymer exhibiting this effective T_{g} is low enough that the mechanical properties are not significantly impacted. In other embodiments, the low effective T_{g}, that is one lower than the highest temperature, or average temperature, encountered in either the ethylene oxide sterilization or the crimping operation, is counterbalanced by both the crystalline polymer domains as well as amorphous polymer domains exhibiting a T_{g} above the temperature of the highest temperature, or average temperature, encountered in either the ethylene oxide sterilization or the crimping operation.

In still other embodiments both the increase in T_{g} due to the inclusion of crystalline domains which act as physical cross-links, along with the reduction in water uptake and/or reduction in uptake of ethylene oxide during the sterilization, may result in a coating which does not lose mechanical integrity, or does not lose substantial mechanical integrity, after crimping and ethylene oxide sterilization. In some embodiments, the mechanical integrity may be better for a coating including a polymer blend of the one of the various embodiments of the present invention compared to a coating utilizing the amorphous polymer of the aforementioned polymer blend alone. In some embodiments, the mechanical integrity of the coating is determined from the observation of SEM images.

In the various embodiments, the size of the crystalline polymer domains may vary. In some embodiments, the crystalline polymer domains may be of essentially, or substantially, uniform size, while in other embodiments, the crystalline domains may vary widely in size such that some domains are approximately 10 times, approximately 8 times, or approximately 5 times the domain size that represents the median size of the crystalline domains (determined by a number average). In some embodiments, the size distribution of the crystalline domains may be high. In some regions, the polydispersity of the crystalline region sizes may be low while in other embodiments the polydispersity may be high.

In some embodiments of the present invention, the coating including the polymer blend composition, includes a large number of small domains of crystallinity, as opposed to a few large crystalline domains. Thus, in some embodiments, the crystalline domains may be uniformly, or substantially uniformly, distributed throughout a coating which includes the polymer blend composition. In other embodiments, the polymer crystallinity may be non-uniformly distributed through the polymer composition, or through the coating that includes the polymer blend composition. In some embodiments, the polymer crystalline domains may be distributed in a manner that prevents cracking of the coating. It is believed that a larger number of smaller crystalline domains that are more or less uniformly distributed will best prevent cracking. It is believed that a larger number of uniformly, or approximately uniformly, distributed smaller crystalline domains is preferable to smaller number of larger domains and/or a less uniform distribution of domains.

In some embodiments, the weight % polymer crystallinity may be about 0.1% to about 50% of the coating. In other embodiments, the weight % polymer crystallinity may be from about 0.5% to about 50%, about 0.5% to about 40%, about 0.5% to about 35%, about 0.5% to about 30%, about 0.5% to about 25%, about 0.5% to about 20%, about 1% to about 35%, about 1% to about 30%, about 1% to about 25%, from about 2% to about 20%, about 2% to about 35%, about 2% to about 30%, about 2% to about 25%, from about 3% to about 18%, or from about 5% to about 15% of the coating. In still other embodiments, the weight % polymer crystallinity may be from about 15% to about 25%, or about 10% to about 45%, or about 10% to about 35%, or in other embodiments from about 3% to about 12% of the coating.

In still other embodiments, the crystalline polymer domains or phase may be measured as a weight percent of the polymer in the coating, rather than the weight percent of the coating. Thus in some embodiments, about 0.1% to about 50% by weight of the polymer in the coating may be crystalline polymer domains. In other embodiments, the weight % polymer crystallinity may be from about 0.5% to about 50%, about 0.5% to about 40%, about 0.5% to about 35%, about 0.5% to about 30%, about 0.5% to about 25%, about 0.5% to about 20%, about 1% to about 35%, about 1% to about 30%, about 1% to about 25%, from about 2% to about 20%, about 2% to about 35%, about 2% to about 30%, about 2% to about 25%, from about 3% to about 18%, or from about 5% to about 15% of the total polymer, which is part of the coating. In still other embodiments, the weight % polymer crystallinity may be from about 15% to about 25%, or about 10% to about 45%, or about 10% to about 35%, or in other embodiments from about 3% to about 12% of the total polymer which is part of the coating. In the aforementioned embodiments, the total weight of the polymer is determined as the sum of all polymers, both semi-crystalline and amorphous polymers, in the coating.

The total amount of crystallinity in the coating, or the polymer blend composition, is a function of the % crystallinity in the semi-crystalline polymer, and the % of the coating that is semi-crystalline polymer, or the % crystallinity in the semi-crystalline polymer and the % total polymer that is semi-crystalline polymer. Thus, the % crystallinity in the semi-crystalline polymer may vary from 20%, 30%, 40%, 50%, 60%, 70%, 80%, 90%, 95%, to 99%.

The % crystallinity may vary with the size of the crystalline domains. In some embodiments, the crystalline domains may be such that there are no, or very few, crystalline domains touching, or adjacent to, each other. In some embodiments, the combination of the size of the crystalline domains, the distribution of the crystalline domains, and the weight percent polymer crystallinity in the coating may be such that the crystalline phase is below the percolation limit or threshold, and therefore, the crystalline domains do not form a continuous phase. In other embodiments, the weight percent polymer crystallinity in the coating may be chosen such that the crystalline phase is above the percolation limit or threshold.

In some embodiments, the polymer crystalline domains may exhibit a Tₘ of greater than the highest temperatures encountered in the sterilization, or that exceeds the temperature encountered in the sterilization procedure for at least about 80%, at least about 70%, at least about 60%, or at least about 50%, of the time period of the sterilization. Thus, in some embodiments, the Tₘ of the polymer crystalline domains may be about 65 °C or higher, about 70 °C or higher, about 75 °C or higher, about 80 °C or higher, about 85 °C or higher, about 90 °C or higher, about 95 °C or higher, about 100 °C or higher, about 110 °C or higher, or about 120 °C or higher.

In other embodiments, high crystallinity may allow for a larger decrease in the effective T_{g}. In some embodiments, the decrease in effective T_{g} may be about 30 °C or more, and/or the effective T_{g} may be below the highest temperature encountered in either the ethylene oxide sterilization or the crimping operation, but the combination of the weight percent of polymer crystallinity in light of the size of the polymer crystalline domains is above the percolation threshold (approximately 15% to 20% or greater), and the crystalline domains have a Tₘ of greater than the highest temperature encountered in the sterilization, and/or that exceeds the temperature encountered in the crimping operation. Thus, the crystalline region is continuous, or semi-continuous, and this limits the impact of the decrease in the T_{g} of the amorphous regions as well as limiting the total uptake of water and/or ethylene oxide due to the lower diffusivity in the crystalline regions along with the increased time needed to dissolve these crystalline regions.

Due to impact of the water and/or ethylene oxide on T_{g}, the water and/or ethylene oxide uptake may impact the mechanical properties. In some embodiments, the polymer blend used for the coating may be chosen to limit uptake of water and/or ethylene oxide such that the dynamic shear storage modulus, G', is at least a factor of about 10, at least a factor of about 8, at least a factor of about 6, at least a factor of about 4, or at least a factor of about 2, greater than the dynamic shear loss modulus, G", when both are measured in the linear viscoelastic range, and at an oscillation frequency of 0.01, 0.1, 1, 10, or 100 radians/second. In some embodiments, the uptake of water and/or ethylene oxide may be limited such that G" is about 2 x 10⁴ Pa or lower when measured at oscillation frequency of 0.01, 0.1, or 1 radians/second during the ethylene oxide sterilization procedure or conditions simulating the impact of the sterilization (temperature and plasticization are comparable to that encountered in sterilization). In some embodiments, the combination of the impact of the crystallinity on the mechanical properties, along with the impact of the crystallinity on the uptake of ethylene oxide and/or water, may result in a value of G' of about 7 x 10⁴ Pa or greater, about 8 x 10⁴ Pa or greater, about 9 x 10⁴ Pa or greater, about 1 x 10⁵ Pa or greater, about 1.5 x 10⁵ Pa or greater, or about 2 x 10⁵ Pa or greater.

In some embodiments, the polymer used for the coating may be chosen to limit uptake of water and/or ethylene oxide such that the dynamic shear storage modulus, G', measured in the linear viscoelastic range at 0.01 radians/second and under the conditions of plasticization and temperature encountered in the ethylene oxide sterilization procedure, is in the rubbery or extended plateau region of the plot of G' versus frequency.

The semi-crystalline polymer may be poly(L-lactic acid) (PLLA). Poly(L-lactic acid) has a T_{g} of approximately 55°C and a Tₘ of 140°C. The crystallinity of PLLA varies from a few % to approximately 65%, and PLLA is generally considered hydrophobic. Other polymers include poly(D-lactic acid) (PDLA). Poly(D-lactic acid) has a T_{g} of approximately 55°C and a Tₘ of 140°C. The crystallinity of PDLA varies from a few % to approximately 65%, and PDLA is also generally considered hydrophobic. Other polymers include poly (L-Lactide-Glycolide) copolymer (PLLGA) comprising at least 70% L-lactide. A specific example would be PLLGA 82/18 containing 82 molar % of L-lactide and 18% molar% of glycolide. Alternatively, poly (D,L-Lactide-Glycolide) copolymer (PLGA) 82/18 could be used. Another semi-crystalline polymer that may be used is poly(L-lactide-glycolide-caprolactone) (PLLA-GA-CL).

The amorphous polymer, or substantially amorphous polymer, is poly (D,L-Lactide-Glycolide) copolymer (PLGA), block co-polymers of polyethylene glycol and poly(D,L-lactic acid) (PEG-PLA), block co-polymers of polyethylene glycol and poly(lactide-co-glycolic acid) (PEG-PLGA), and poly(D,L-lactide-glycolide-caprolactone) terpolymers. For amorphous poly (D,L-Lactide-Glycolide) copolymer (PLGA), the molar ratio of D,L-lactide to glycolide may range from 10:90, 20:80, 25:75, 40:60, 50:50, 60:50, 75:25, 80:20, or 90:10.

A particularly preferred amorphous polymers class is the class of poly(lactide-glycolide-caprolactone) terpolymers. These polymers are amorphous, or substantially amorphous, and have T_{g} in the range of -40 to 50°C. The copolymers are hydrophobic.

In all the polymers comprising both D-lactide and L-lactide the ratio of the two diastereomers could vary from 0-100, being for example 0.10, 0.50, 1, 5, 10, 20, 30, 40, 50, 60, 70, 80, 90, 95, 99, 99.5, 99.9 or any other ratio. In any of the previously mentioned polymers the D- or the L-lactide could alternatively be replaced with meso-lactide.

According to the invention, the weight-average-molecular-weight range is 75,000 to 300,000, from about 75,000 to about 150,000, from about 100,000 to about 200,000, from about 100,000 to about 250,000, from about 150,000 to about 300,000, from about 200,000 to about 300,000, or from about 200,000 to about 350,000, for both the semi-crystalline and the amorphous, or substantially amorphous, polymers.

In the various embodiments of the present invention, the weight % of semi-crystalline polymer in the polymer blend that is utilized in a coating can range from about 5% to about 75%. In some embodiments, the weight % of semi-crystalline to the total polymer, that is the sum of all amorphous polymer and all semi-crystalline polymer, may range from about 5% to about 25%, about 5% to about 20%, about 10% to about 20%, from about 20% to about 30%, from about 30% to about 50%, from about 45% to about 75%, and from about 50% to about 75%.

In the various embodiments, the sum of the polymer blend and the drug, may be, by weight % of the coating, about 10% to about 100%, about 20% to about 100%, about 20% to about 90%, about 20% to about 80%, about 30% to about 100%, about 30% to about 90%, about 30% to about 80%, about 40% to about 100%, about 40% to about 90%, about 40% to about 80%, about 50% to about 100%, about 50% to about 90%, about 50% to about 80%, about 60% to about 100%, about 60% to about 90%, about 60% to about 80%, about 70% to about 100%, about 70% to about 90%, about 70% to about 80%, about 80% to about 100%, about 80% to about 95%, about 90% to about 90%, about 90% to about 100%, and about 95% to about 100%.

As discussed below, active agents may also be added to the coating. The ratio of drug to the total polymer, that is the sum of all of the amorphous and all of the semi-crystalline polymer, may range from about 1:1 to about 1:5. In some embodiments, the ratio of drug to polymer may be in between these ratios, or the drug to polymer ratio may be about 1:2, about 1:3, or about 1:4, or any ratio between these specified ratios. In some embodiments, the dose of drug may be low such that the drug to polymer ratio is lower than about 1:5 such as about 1:7, about 1:8 or about 1:10, or lower. In other cases, the drug to polymer ratio may be larger than 1:1, that is up to about 4:3 or 3:2 or higher.

### Active Agents

In some embodiments, the implantable device described herein can optionally include at least one bioactive agent. An implantable device, such as a stent, can be designed for the localized delivery of a therapeutic agent. A medicated implantable device may be constructed in part, e.g., by coating the device with a coating material containing an active agent. The body of the device may also contain an active agent.

Examples of suitable bioactive agents include, but are not limited to, synthetic inorganic and organic compounds, proteins and peptides, polysaccharides and other sugars, lipids, and DNA and RNA nucleic acid sequences having therapeutic, prophylactic or diagnostic activities. Nucleic acid sequences include genes, antisense molecules that bind to complementary DNA to inhibit transcription, and ribozymes. Some other examples of other bioactive agents include antibodies, receptor ligands, enzymes, adhesion peptides, blood clotting factors, inhibitors or clot dissolving agents such as streptokinase and tissue plasminogen activator, antigens for immunization, hormones and growth factors, oligonucleotides such as antisense oligonucleotides and ribozymes and retroviral vectors for use in gene therapy.

In certain embodiments, optionally in combination with one or more other embodiments described herein, the implantable device can include at least one bioactive agent selected from antiproliferative, antineoplastic, antimitotic, anti-inflammatory, antiplatelet, anticoagulant, antifibrin, antithrombin, antibiotic, antiallergic and antioxidant substances. An anti-inflammatory drug can be a steroidal anti-inflammatory drug, a nonsteroidal anti-inflammatory drug (NSAID), or a combination of a steroidal and a nonsteroidal anti-inflammatory may be used.

In addition, the bioactive agents can be other than antiproliferative or anti-inflammatory agents. In some embodiments, such other agents can be used in combination with antiproliferative or anti-inflammatory agents, and/or one or more of each of the anti-inflammatory and antiproliferative may be included in a device. These other bioactive agents can have other properties such as antineoptastic, antimitotic, cystostatic, antiplatelet, anticoagulant, antifibrin, antithrombin, antibiotic, antiallergic, and/or antioxidant properties.

Other bioactive agents that can be used include alpha-interferon, genetically engineered epithelial cells, and dexamethasone. Another type of active agent is a "prohealing" drug or agent, which in the context of a blood-contacting implantable device, refers to a drug or agent that has the property that it promotes or enhances re-endothelialization of arterial lumen to promote healing of the vascular tissue.

In a more specific embodiment, optionally in combination with one or more other embodiments described herein, the implantable device, or coating, of the invention comprises at least one bioactive agent selected from paclitaxel, docetaxel, estradiol, nitric oxide donors, super oxide dismutases, super oxide dismutase mimics, 4-amino-2,2,6,6-tetramethylpiperidine-1-oxyl (4-amino-TEMPO), tacrolimus, dexamethasone, rapamycin, rapamycin derivatives, 40-*O*-(2-hydroxy)ethyl-rapamycin (everolimus), 40-*O*-(2-ethoxy)ethyl-rapamycin (biolimus), 40-*O*-(3-hydroxy)propyl-rapamycin, 40-*O*-[2-(2-hydroxy)ethoxy]ethyl-rapamycin, 40-*O*-tetrazole-rapamycin, 40-epi-(N1-tetrazolyl)-rapamycin (zotarolimus), pimecrolimus, imatinib mesylate, midostaurin, clobetasol, dexamethasone, dexamethasone acetate, fenofibric acid, fenofibrate, progenitor cell-capturing antibodies, prohealing drugs, cRGD, prodrugs thereof, co-drugs thereof, and a combination thereof. In a particular embodiment, the bioactive agent is everolimus. In another specific embodiment, the bioactive agent is zotarolimus. In another specific embodiment, the bioactive agent is a limus drug combined with another type of drug. In another specific embodiment, the bioactive agent is a limus drug combined with an anti-inflammatory drug. A "limus drug" is a drug included in the group of sirolimus and its derivatives, such as but not limited to, everolimus, tacrolimus, biolimus, and zotarolimus.

The foregoing bioactive agents are listed by way of example and are not meant to be limiting. Other bioactive agents that are currently available or that may be developed in the future are equally applicable.

### Coating construct

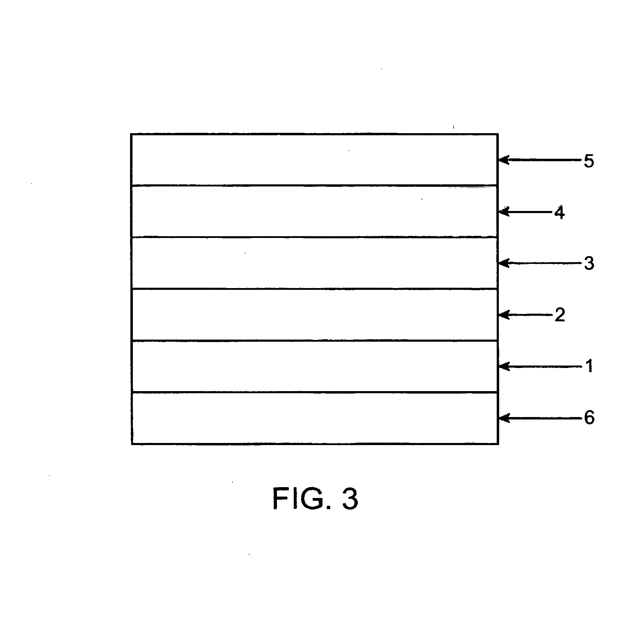

In some embodiments, a coating including the polymer blend composition is disposed over an implantable device (e.g., a stent) as described herein in a layer according to any design of a coating. FIG. 3 is a pictorial representation of multiple coatings on a substrate. The coating can be a multi-layer structure that includes, with reference to FIG. 3, at least one reservoir layer (2), and optionally any one or more of the following, a primer layer (1) which is applied directly onto the substrate (6) and is below the reservoir layer, and a release control layer (3) on top of the reservoir layer, with an optional topcoat layer (4) and an optional finishing layer (5).

The reservoir layer is also referred to as a "matrix layer" or "drug matrix" can be a drug-polymer layer including at least one polymer (drug-polymer layer) or, alternatively, a polymer-free drug layer. In some embodiments, a coating of the invention can include two or more reservoir layers described above, each of which can include an active agent described herein. Similarly, in some embodiments there may be more than one primer layer, topcoat layer, release control layer, and/or finishing layer. In some embodiments there are additional coating layers not specifically labeled or identified as above. For example two or more reservoir layers each including a drug may be separated by an intervening layer to limit interaction between the two drugs during manufacturing and storage.

Each layer of a stent coating can be disposed over the implantable device (e.g., a stent) by dissolving or dispersing the polymer blend composition, optionally with one or more other polymers and/or other additives, in a solvent, or a mixture of solvents (where the solvent is a liquid or fluid), and disposing the resulting coating solution over the stent by procedures such as spraying or immersing the stent in the solution. Such coating procedures are well-known in the art. After the solution has been disposed over the stent, the solvent is removed, or substantially removed, by evaporation. When the solvent is removed, what is essentially left, or left, is the solid material which forms a layer, film, or coating on the surface of the implantable medical device, either directly or indirectly. The process of drying can be accelerated if the drying is conducted at an elevated temperature, and/or with the addition of a flow of air, another gas or fluid, over or past the device to enhance mass transfer of the solvent.

The complete implantable medical device (such as a stent) coating may be optionally annealed at a temperature between about 40 °C and about 150 °C for a period of time between about 5 minutes and about 180 minutes, if desired, to allow for crystallization of the polymer coating, and/or to improve the thermodynamic long term stability of the coating.

To incorporate a bioactive agent (e.g., a drug) into the reservoir layer, the drug can be combined with the polymer solution or dispersion, or a solution or dispersion of the drug in solvent can be prepared, and then the solution or dispersion of drug, optionally including polymer, is disposed over the implantable device as described above.

The drug-polymer layer can be applied directly or indirectly over at least a portion of the stent surface to serve as a reservoir for at least one bioactive agent (e.g., drug) that is incorporated into the reservoir layer. The optional primer layer can be applied between the stent and the reservoir to improve the adhesion of the drug-polymer layer to the stent. The optional topcoat layer can be applied over at least a portion of the reservoir layer and may serve as a rate-limiting membrane that helps to control the rate of release of the drug. In one embodiment, the topcoat layer can be essentially free from any bioactive agents or drugs. If the topcoat layer is used, the optional finishing coat layer can be applied over at least a portion of the topcoat layer for further control of the drug-release rate and for improving the biocompatibility of the coating. Without the topcoat layer, the finishing coat layer can be deposited directly on the reservoir layer. Any of the layers may optionally include one or more drugs.

In some embodiments, the drug-polymer layer, or any other layer described herein, may cover all, or substantially all, of the surface of the implantable medical device. That is the surface, whether bare or previously coated, is coated or covered completely, or essentially completely. In other embodiments, only some portion of the surface, such as about 30%, about 50%, about 60%, about 70% or about 80%, may be coated. In some embodiments, a portion of the device's surface may be selectively coated (directly or indirectly) such as an abluminal surface may be selectively coated, or a luminal surface may be selectively coated.

The various embodiments of the present invention include a coating including a polymer blend composition having a range of thickness over an implantable device. In certain embodiments, the coating that is deposited over at least a portion of the implantable device has a thickness of ≤ about 30 micron, or ≤ about 20 micron, or ≤ about 10 micron, or ≤ about 5 micron, or ≤ about 3 micron. These dimensions apply to each of the individual layers if more than one layer is deposited on the surface of the medical device, either directly or indirectly.

The drug can be released by virtue of the degradation, dissolution, and/or erosion of the layer(s) forming the coating, or via migration (diffusion) of the drug through the polymer blend composition in the layer(s) into a blood vessel or tissue. The active agent may be released by any number of mechanisms, including, but not limited to, any one, or any combination of the above mentioned release mechanisms, and in addition, may be released by other mechanisms not specifically mentioned, but known in the art, such as, but not limited to, release due to osmotic effects.

In one embodiment, any or all of the layers of the implantable medical device coating, or stent coating, may be made of a coating including a polymer blend composition as described herein. In some embodiments the layer may have the property of being biologically degradable/erodable/ absorbable/ resorbable, while in other embodiments, the layer may have the property of being non-degradable/biostable polymer. In some embodiments, the layer may include polymers or other materials that are degradable as well as some polymer and/or materials that are biostable. In another embodiment, the outermost layer of the coating may be limited to a coating including a polymer blend composition, that is including an embodiment of the polymer blend composition as defined above. In some embodiments, the polymer(s) in a particular layer may be the same as, or different than, those in any of the other layers. In some embodiments, the layer on the outside of another bioabsorbable may also be bioabsorbable and degrade at a similar or faster relative to the inner layer. In some embodiments, the inner layer may be bioabsorbable, and the layer on the outside may be biodegradable and degrade at a similar or slower rate and, in such embodiments, the products of the degradation of the inner layer may diffuse through the outside layer. Any layer of a coating can contain any amount of a polymer blend composition as described herein. In some embodiments, any layer of a stent coating may also contain any amount of a non-degradable polymer, or a blend of more than one such polymer, but it may be that neither the non-degradable polymer is mixed with a bioabsorbable polymer, nor any layer underneath the non-degradable layer includes a bioabsorbable polymer. In other embodiments, a layer may include both non-degradable and degradable polymer, but in such quantities that the products of the degraded polymer can diffuse through the remaining non-degradable polymer in the layer, and/or diffuse through any layers on top of the given layer. In some embodiments, a bioabsorbable polymer and/or layer may be below a non-degradable layer, and/or below a layer including both degradable and non-degradable polymer or material, and the products of the degradation of the degradable polymer diffuse through the non-degradable layer.

Non-limiting examples of bioabsorbable polymers and biocompatible polymers include poly(N-vinyl pyrrolidone); polydioxanone; polyorthoesters; polyanhydrides; poly(glycolic acid); poly(glycolic acid-co-trimethylene carbonate); polyphosphoesters; polyphosphoester urethanes; poly(amino acids); poly(trimethylene carbonate); poly(iminocarbonates); co-poly(ether-esters); polyalkylene oxalates; polyphosphazenes; biomolecules; e.g., fibrin, fibrinogen, cellulose, cellophane, starch, collagen, hyaluronic acid, and derivatives thereof (e.g., cellulose acetate, cellulose butyrate, cellulose acetate butyrate, cellulose nitrate, cellulose propionate, cellulose ethers, and carboxymethyl cellulose), polyurethane, polyesters, polycarbonates, polyurethanes, poly(L-lactic acid-co-caprolactone) (PLLA-CL), poly(D-lactic acid-co-caprolactone) (PDLA-CL), poly(D,L-lactic acid-co-caprolactone) (PDLLA-CL), poly(D,L-lactic acid-glycolic acid")" (PLGA), poly(L-lactic acid-glycolic acid) (PLLGA), poly(D-lactic acid-co-glycolide-co-caprolactone) (PDLA-GA-CL), poly(L-lactic acid-co-glycolide-co-caprolactone) (PLLA-GA-CL), poly(D,L-lactic acid-co-glycolide-co-caprolactone) (PDLLA-GA-CL), poly(glycolide-co-caprolactone) (PGA-CL), or any copolymers thereof, or any combinations thereof.

Non-limiting examples of non-degradable polymers include methylmethacrylate, ethylmethacrylate, butylmethacrylate, 2-ethylhexylmethacrylate, laurylmethacrylate, hydroxyl ethyl methacrylate, polyethylene glycol (PEG) acrylate, PEG methacrylate, 2-methacryloyloxyethylphosphorylcholine (MPC) and n-vinyl pyrrolidone, methacrylic acid, acrylic acid, hydroxypropyl methacrylate, hydroxypropylmethacrylamide, 3-trimethylsilylpropyl methacrylate, and copolymers thereof, or any combinations thereof.

Any copolymer, whether random, graft, or block copolymers, including any one or more of the polymers in the above list (and/or constituent monomers of the polymers in the above list), regardless of which other polymer, polymers, or monomers comprise the copolymer, and without regard for whether or not the other polymer, polymers or monomers are specifically listed herein, is also encompassed in the current invention. Various embodiments of the current invention also include cross-linked and uncross-linked polymers.

All embodiments may also includes additional components such as, but not limited to lubricating agents, fillers, plasticizing agents, surfactants, diluents, mold release agents, agents which act as active agent carriers or binders, anti-tack agents, anti-foaming agents, viscosity modifiers, anti-oxidants, potentially residual levels of solvents, and potentially any other agent which aids in, or is desirable in, the processing of the material, and/or is useful or desirable as a component of the final product. Surfactants may be used for the preparation of a dispersion of polymer and/or drug in a solvent or fluid.

### Method of Treating or Preventing Disorders

An implantable device according to the present invention can be used to treat, prevent, mitigate, reduce, or diagnose various conditions or disorders, or to provide a pro-heating effect. Examples of such conditions or disorders include, but are not limited to, atherosclerosis, thrombosis, restenosis, hemorrhage, vascular dissection, vascular perforation, vascular aneurysm, vulnerable plaque, chronic total occlusion, patent foramen ovale, claudication, anastomotic proliferation of vein and artificial grafts, arteriovenous anastamoses, bile duct obstruction, ureter obstruction and tumor obstruction. In some embodiments, the vascular medical condition or vascular condition is a coronary artery disease (CAD) and/or a peripheral vascular disease (PVD).

A portion of the implantable device or the whole device itself can be formed of the material, that is the polymer blend composition which optionally includes one or more active agents, as described herein. For example, the material can be a coating disposed over at least a portion of the device.

Although various embodiments of the invention have specified a stent, the coating may be applied to implantable medical devices in general, such as but not limited to, stents, grafts, stem-grafts, catheters, leads and electrodes, clips, shunts, closure devices, valves, and particles. Other particular applications include temporary occlusive devices, sealants, and grafts, or any medical device in which a polymeric coating is necessary, useful, or advantageous. With respect to any reference to stent, the stents may be intended for any vessel in the body, including neurological, carotid, vein graft, coronary, aortic, renal, iliac, femoral, popliteal vasculature, and urethral passages.

### EXAMPLES

The examples set forth below are for illustrative purposes only and are in no way meant to limit the invention. The following examples are given to aid in understanding the invention, but it is to be understood that the invention is not limited to the particular materials or procedures of examples.

### Example 1:

For a desired dose per surface area of 100 ug/cm³ of Everolimus for a small (12 mm) VISION™ stent (Advanced Cardiovascular Systems) the following coatings are prepared. The first step was to coat the exterior of the stent with approximately 55µg of a primer which was PLGA amorphous polymer with a molar ratio of lactide to glycolide of 75/25, and was sprayed on from a solution (2% by weight solids) of a mixture of acetone and methyl-isobutyl ketone at a 9:1 ratio. The stent was cured, or placed in an oven, at 140 °C for 30 minutes. Then, PLGA 50/50 is blended with semi-crystalline PLLA at a 90:10 weight:weight ratio in chloroform. To the polymer blend in solvent, the drug, Everolimus, was added. The 1% by weight PLGA/PLLA/Everolimus solution (or dispersion) was sprayed onto the stent, and the solvent removed. The amount of additional material deposited onto the stent was approximately 112 µg of which about 56 µg was drug, Everolimus, and the balance is the polymer blend composition. The stent was then baked at 50 °C for 2 hours. The over all thickness is estimated to be approximately 3- 4 µm. After crimping the stent onto a catheter balloon for delivery, and placing the entire assembly in the final packaging, the stent was sterilized in ethylene oxide. SEM was performed after a use test, which consists of placing the stent in a simulated artery, expanding the stent to nominal diameter, and subjecting the stent to flow of PBS, deionized water, or serum at 37°C for 1 hour. PBS is a buffer, phosphate buffer saline, commonly used in biochemistry, and which contains sodium chloride, sodium phosphate, and potassium phosphate. The concentration usually is isotonic with the human body.

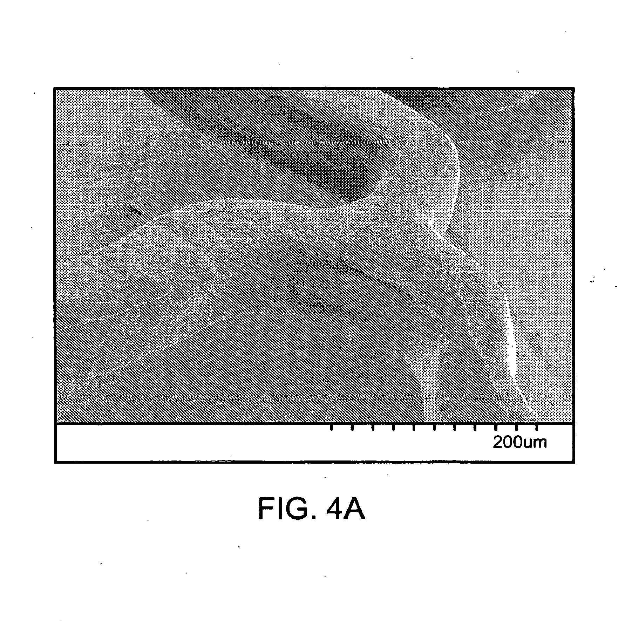

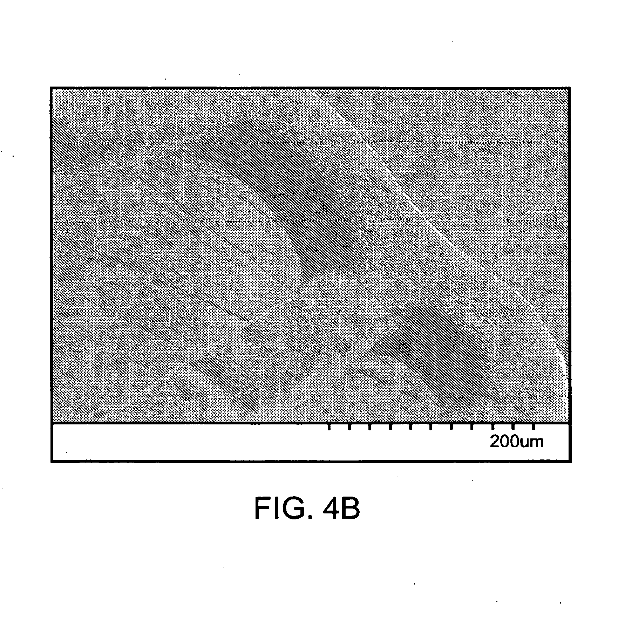

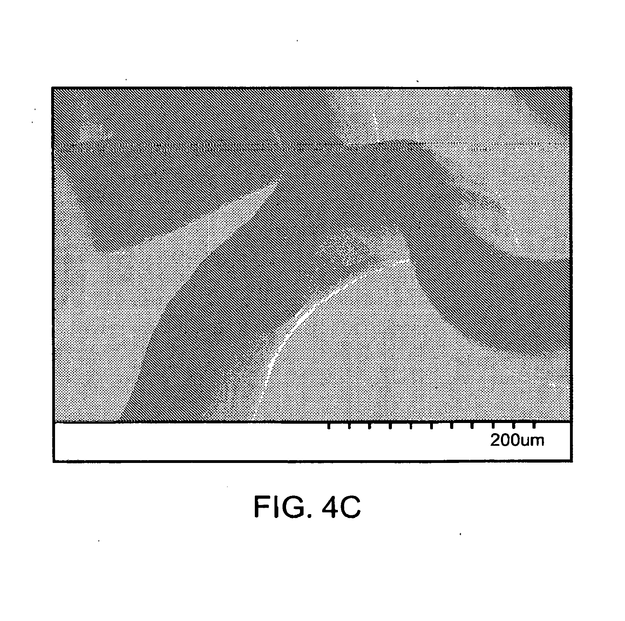

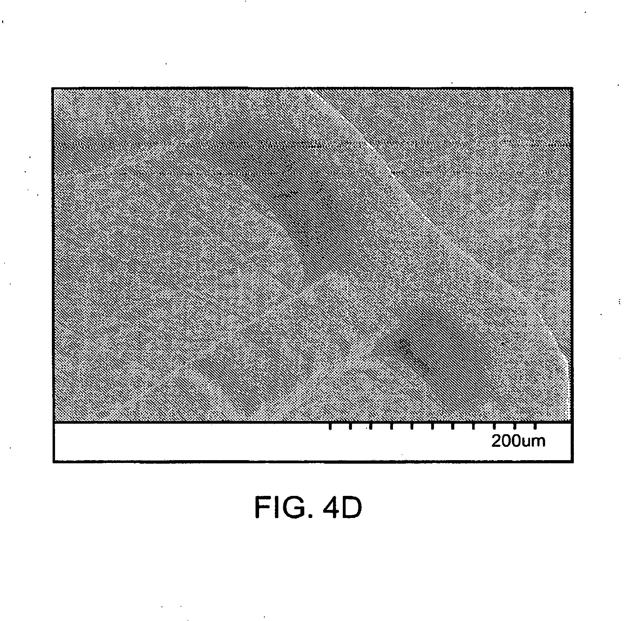

FIGs. 4A - 4D are SEM images of the coating after sterilization. As seen in comparing FIGs. 4A - 4D with FIGs. 2A - 2D, the use of one of the embodiments of the polymer blend of the present invention improves the appearance of the coating.

### Example 2:

For a desired dose per surface area of 100 ug/cm³ of Everolimus for a small (12 mm) VISION™ stent (Advanced Cardiovascular Systems) the following coatings are prepared. The first step was to coat the exterior of the stent with approximately 55µg of a primer which was PLGA amorphous polymer with a molar ratio of lactide to glycolide of 75/25 and was sprayed on from a solution of a mixture of acetone and methyl-isobutyl ketone at a 9:1 ratio. The stent was cured, or placed in an oven, at 140 °C for 30 minutes. Then, PLGA 75/25 was blended with semi-crystalline PLLA at a 90:10 weight:weight ratio in chloroform. To the polymer blend in solvent, the drug, Everolimus, was added. The 1% by weight PLGA/PLLA/Everolimus solution (or dispersion) was sprayed onto the stent, and the solvent removed. The amount of additional material deposited onto the stent was approximately 112 µg of which about 56 µg was drug, Everolimus, and the balance is the polymer blend composition. The stent is then baked at 50 °C for 2 hours. The thickness is estimated to be approximately 3-4 µm. After crimping the stent onto a catheter balloon for delivery, and placing the entire assembly in the final packaging, the stent was sterilized in ethylene oxide. SEM was performed after as use test (described above in Example 1).

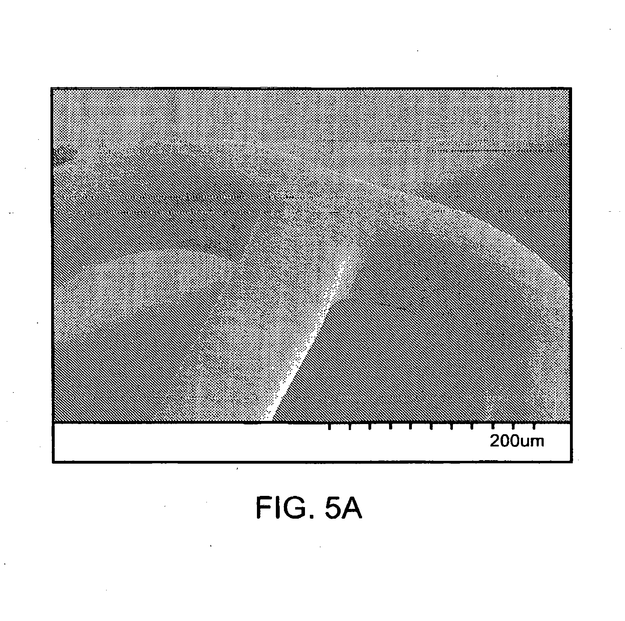

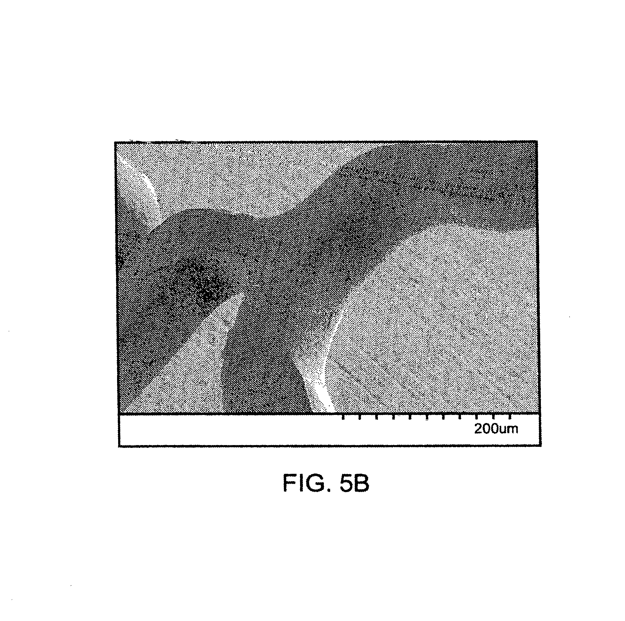

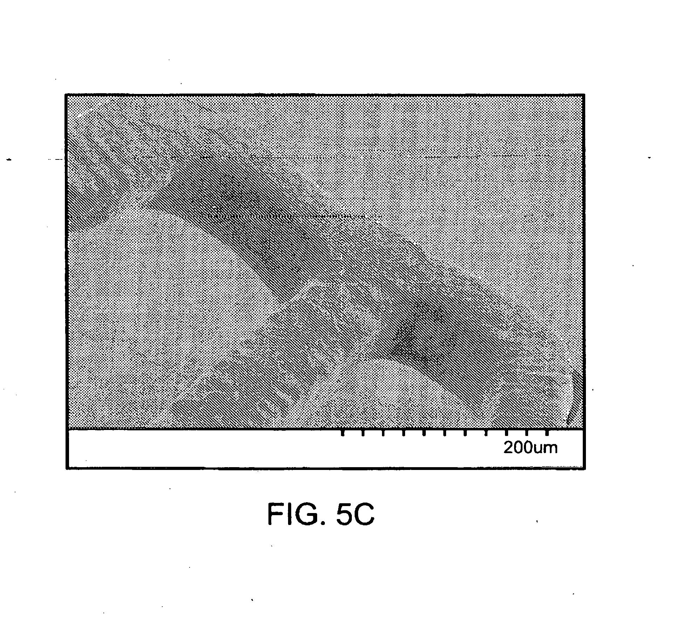

Figures 5A - 5C are SEM images of the coating after sterilization. As seen in comparing FIGs. 5A - 5C with FIGs. 2A - 2D, the use of one of the embodiments of the polymer blend of the present invention improves the appearance of the coating.

### Example 3 (prospective example of coating preparation):

This prospective example is an illustration of how to formulate an exemplary embodiment of a coating of the present invention.

For a desired dose per surface area of 100 ug/cm³ of Everolimus for a small (12 mm) VISION™ stent (Advanced Cardiovascular Systems) the following coatings are prepared. The first step is to coat the exterior of the stent with approximately 55µg of a primer which is PLGA amorphous polymer with a molar ratio of lactide to glycolide of 75/25 and is sprayed on from a solution of a mixture of acetone and methyl-isobutyl ketone at a 9:1 ratio. The stent is cured, or placed in an oven, at 140 °C for 30 minutes. Then, PLGA 50/50 is blended with semi-crystalline PLLA at a 75/25 weight (PLLA):weight(PLGA) ratio in chloroform. To the polymer blend in solvent, the drug, Everolimus, is added. The solution is sprayed onto the stent. The amount of additional material deposited onto the stent is approximately 112 µg of which about 56 µg is drug, Everolimus, and the balance is the polymer blend composition. The stent is then baked at 50 °C for 2 hours. The thickness is estimated to be approximately 3 µm.

### Example 4 (prospective example of coating preparation):

This prospective example is an illustration of how to formulate an exemplary embodiment of a coating of the present invention.

For a desired dose per surface area of 100 ug/cm³ of Everulimus for a small (12 mm) VISION™ stent (Advanced Cardiovascular Systems) the following coatings is prepared. The first step is to coat the exterior of the stent with approximately 55µg of a primer which is PLGA amorphous polymer with a molar ratio of lactide to glycolide of 75/25 and is sprayed on from a solution of a mixture of acetone and methyl-isobutyl ketone at a 9:1 ratio. The stent is cured, or placed in an oven, at 140 °C for 30 minutes. Then, PLGA 75/25 is blended with semi-crystalline PLLA at a 50:50 weight:weight ratio in chloroform. To the polymer blend in solvent, the drug, Everolimus, is added. The amount of additional material deposited onto the stent is approximately 168 µg of which about 56 µg is drug, Everolimus, and the balance is the polymer blend composition. The stent is then baked at 50 °C for 2 hours. The thickness is estimated to be approximately 4 µm.

### Example 5 (prospective example of coating preparation):

This prospective example is an illustration of how to formulate an exemplary embodiment of a coating of the present invention.

For a desired dose per surface area of 100 ug/cm³ of Everolimus for a small (12 mm) VISION™ stent (Advanced Cardiovascular Systems) the following coatings are prepared. The first step is to coat the exterior of the stent with approximately 55µg of a primer which is PLGA amorphous polymer with a molar ratio of lactide to glycolide of 75/25 and is sprayed on from a solution of a mixture of acetone and methyl-isobutyl ketone at a 9:1 ratio. The stent is cured, or placed in an oven, at 140 °C for 30 minutes. Then, PLGA 75/25 is blended with semi-crystalline PLLGA (82/18) at a 50:50 weight:weight ratio in chloroform. To the polymer blend in solvent, the drug, Everolimus, is added. The amount of additional material deposited onto the stent is approximately 168 µg of which about 56 µg is drug, Everolimus, and the balance is the polymer blend composition. The stent is then baked at 50 °C for 2 hours. The thickness is estimated to be approximately 4 µm.

While particular embodiments of the present invention have been shown and described, it will be obvious to those skilled in the art that changes and modifications can be made without departing from this invention in its broader aspects.

## Claims

1. A coating that comprises:
a polymer blend composition, the polymer blend composition comprising:
a semi-crystalline polymer with a weight-average-molecular-weight from 75,000 to 300,000;
an amorphous, or substantially amorphous, polymer with a weight-average-molecular weight from 75,000 to 300,000 and wherein the amorphous, or substantially amorphous, polymer is selected from the group consisting of poly(D,L-lactic acid-co-glycolic acid) (PLGA), polyethylene glycol-block-poly(D,L-lactic acid) (PEG-PLA), polyethylene glycol-block-poly(D,L-lactic acid-co-glycolic acid) (PEG-PLGA), poly(lactide-glycolide-caprolactone)terpolymers, and combinations thereof;
wherein
the semi-crystalline polymer is between 2% and 75% by weight of the sum of the semi-crystalline and the amorphous, or substantially amorphous, polymer;
wherein
the effective glass transition temperature of the coating is - 60 °C or higher;
the melting transition of a crystalline polymer region, or at least one melting transition of a polymer crystalline region if there are more than one polymer melt transitions, is 70 °C or higher;
the coating comprises 0.5% to 50% by weight polymer crystallinity; and the effective glass transition temperature is the measured glass transition temperature before exposure to ethylene oxide sterilization and, if more than one glass transition temperature is observed for the blend, then the effective glass transition temperature is the lower of the two or more distinctly observable glass transition temperature values.

2. The coating of claim 1, wherein the semi-crystalline polymer is selected from the group consisting of poly(D-lactic acid) (PDLA), poly(L-lactic acid) (PLLA), poly(L-lactic acid-co-glycolic acid) (PLLGA), poly(L-lactic acid-co-glycolide-co-caprolactone) (PLLA-GA-CL), and combinations thereof.

3. The coating of claim 1, wherein the amorphous, or substantially amorphous, polymer is poly(D,L-lactic acid-co-glycolic acid) (PLGA).

4. The coating of claim 3, wherein the poly(D,L-lactic acid-co-glycolic acid) (PLGA) is selected from the group consisting of poly(D,L-lactic acid-co-glycolic acid) (PLGA) 50/50, poly(D,L-lactic acid-co-glycolic acid) (PLGA) 75/25, poly(D,L-lactic acid-co-glycolic acid) (PLGA) 90/10, and combinations thereof.

5. The coating of claim 1, wherein the amorphous, or substantially amorphous, polymer is a poly(lactide-glycolide-caprolactone)terpolymer.

6. The coating of claim 1, wherein the coating comprises 1% to 35% by weight polymer crystallinity.

7. The coating of claim 1, wherein the coating comprises 2% to 30% by weight polymer crystallinity.

8. The coating of claim 1, wherein the dynamic shear loss modulus when measured in the linear viscoelastic range at an oscillation frequency of 1 radian/second is 2 X 10⁴ or less.

9. The coating of claim 1, the coating further comprising a drug.

10. The coating of claim 9, the drug selected from the group consisting of everolimus, zotarolimus, dexamethasone, and combinations thereof.

11. An implantable medical device comprising the coating according to claim 1.

12. The device of claim 11, wherein the semi-crystalline polymer in the coating is selected from the group consisting ofpoly(D-lactic acid) (PDLA), poly(L-lactic acid) (PLLA), poly(L-lactic acid-co-glycolic acid) (PLLGA), poly(L-lactic acid-co-glycolide-co-caprolactone) (PLLA-GA-CL), and combinations thereof.

13. The device of claim 11, wherein the amorphous, or substantially amorphous, polymer in the coating is poly(D,L-lactic acid-co-glycolic acid) (PLGA).

14. The device of claim 13, wherein the poly(D,L-lactic acid-co-glycolic acid) (PLGA) is selected from the group consisting of poly(D,L-lactic acid-co-glycolic acid) (PLGA) 50/50, poly(D,L-lactic acid-co-glycolic acid) (PLGA) 75/25, poly(D,L-lactic acid-co-glycolic acid) (PLGA) 90/10, and combinations thereof.

15. The device of claim 11, wherein the amorphous, or substantially amorphous, polymer in the coating is a poly(lactide-glycolide-caprolactone)terpolymer.

16. The device of claim 11, wherein the coating comprises 1% to 35% by weight polymer crystallinity.

17. The device of claim 11, wherein the coating comprises 2% to 30% by weight polymer crystallinity.

18. The device of claim 11, wherein the dynamic shear loss modulus of the coating when measured in the linear viscoelastic range at an oscillation frequency of 1 radian/second is 2 X 10⁴ or less.

19. An implantable medical device comprising a coating:
the coating comprising:
a polymer blend composition, the polymer blend composition comprising:
a semi-crystalline polymer with a weight-average-molecular-weight from 75,000 to 300,000;
an amorphous, or substantially amorphous, polymer with a weight-average-molecular weight from 75,000 to 300,000;
wherein
the semi-crystalline polymer is between 2% and 75% by weight of the sum of the semi-crystalline and the amorphous, or substantially amorphous, polymer;
the semi-crystalline polymer is selected from the group consisting of poly(D-lactic acid) (PDLA), poly(L-lactic acid) (PLLA), poly(L-lactic acid-co-glycolic acid) (PLLGA), poly(L-lactic acid-co-glycolide-co- caprolactone) (PLLA-GA-CL), and combinations thereof,
and the amorphous, or substantially amorphous polymer is selected from the group consisting of poly(D,L-lactide-glycolide), poly(lactide-glycolide-caprolactone)terpolymers, and combinations thereof,
wherein
the effective glass transition of the coating is - 60 °C or higher; the coating comprises 0.5% to 50% by weight polymer crystallinity;
the coating has a water content of 10% or less after sterilization; and
the effective glass transition temperature is the measured glass transition temperature before exposure to ethylene oxide sterilization and, if more than one glass transition temperature is observed for the blend, then the effective glass transition temperature is the lower of the two or more distinctly observable glass transition temperature values.

## Patentansprüche

1. Eine Beschichtung, die folgendes umfasst:
eine Polymermischungszusammensetzung, wobei die Polymermischungszusammensetzung folgendes umfasst:
ein teilkristallines Polymer mit einem Gewichtsmittel des Molekulargewichts, das von 75.000 bis 300.000 reicht;
ein amorphes, oder im Wesentlichen amorphes Polymer mit einem Gewichtsmittel des Molekulargewichts, das von 75.000 bis 300.000 reicht und wobei das amorphe oder im Wesentlichen amorphe Polymer ausgewählt ist aus der Gruppe bestehend aus Poly(D,L-Milchsäure-co-Glykolsäure) (PLGA), Polyethylenglycol-Block-Poly(D,L-Milchsäure) (PEG-PLA), Polyethylenglycol-Block-Poly(D,L-Milchsäure-co-Glykolsäure) (PEG-PLGA), Poly(Lactid-Glycolid-Caprolacton)Terpolymeren, und Kombinationen davon;
wobei
das teilkristalline Polymer zwischen 2 Gew.-% und 75 Gew.% der Summe des teilkristallinen und des amorphen oder im Wesentlichen amorphen Polymers entspricht;
wobei
die effektive Glasübergangstemperatur der Beschichtung -60 °C oder höher ist;
der Schmelzübergang eines Bereichs des kristallinen Polymers, oder mindestens ein Schmelzübergang eines Bereichs des kristallinen Polymers, wenn es mehr als einen Polymerschmelzübergang gibt, 70 °C oder höher ist;die Beschichtung 0,5 Gew.-% bis 50 Gew.-% Polymerkristallinität umfasst, und die effektive Glasübergangstemperatur die gemessene Glasübergangstemperatur vor der Exposition gegenüber Ethylenoxid-Sterilisation ist und, wenn mehr als eine Glasübergangstemperatur für die Mischung beobachtet wird, dann ist die effektive Glasübergangstemperatur die geringste der zwei oder mehreren deutlich beobachtbare Glasübergangstemperaturwerten.

2. Die Beschichtung des Anspruchs 1, wobei das teilkristalline Polymer ausgewählt aus der Gruppe bestehend aus Poly(D-Milchsäure) (PDLA), Poly(L-Milchsäure) (PLLA), Poly(L-Milchsäure-co-Glykolsäure) (PLLGA), Poly(L-Milchsäure-co-Glykolid-co-Caprolacton) (PLLA-GA-CL), und Kombinationen davon ist.

3. Die Beschichtung des Anspruchs 1, wobei das amorphe, oder im Wesentlichen amorphe Polymer Poly(D,L-Milchsäure-co-Glykolsäure) (PLGA) ist.

4. Die Beschichtung des Anspruchs 3, wobei das Poly(D,L-Milchsäure-co-Glykolsäure) (PLGA) ausgewählt aus der Gruppe bestehend aus Poly(D,L-Milchsäure-co-Glykolsäure) (PLGA) 50/50, Poly(D,L-Milchsäure-co-Glykolsäure) (PLGA) 75/25, Poly(D,L-Milchsäure-co-Glykolsäure) (PLGA) 90/10, und Kombinationen davon ist.

5. Die Beschichtung des Anspruchs 1, wobei das amorphe, oder im Wesentlichen amorphe Polymer ein Poly(Lactid-Glycolid-Caprolacton)Terpolymer ist.

6. Die Beschichtung des Anspruchs 1, wobei die Beschichtung 1 Gew.-% bis 35 Gew.-% Polymerkristallinität umfasst.

7. Die Beschichtung des Anspruchs 1, wobei die Beschichtung 2 Gew.-% bis 30 Gew.-% Polymerkristallinität umfasst.

8. Die Beschichtung des Anspruchs 1, wobei der dynamichen Verlustmodul gemessen in einem linearen viskoelastischen Bereich bei einer Schwingungsfrequenz von 1 Radiant/Sekunde 2 X 10⁴ oder geringer ist.

9. Die Beschichtung des Anspruchs 1, wobei die Beschichtung weiterhin ein Arzneimittel umfasst.

10. Die Beschichtung des Anspruchs 9, wobei das Arzneimittel ausgewählt aus der Gruppe bestehend aus Everolimus, Zotarolimus, Dexamethason, und Kombinationen davon ist.

11. Eine implantierbare medizinische Vorrichtung umfassend die Beschichtung nach Anspruch 1.

12. Die Vorrichtung des Anspruchs 11, wobei das teilkristalline Polymer der Beschichtung ausgewählt aus der Gruppe bestehend aus Poly(D-Milchsäure) (PDLA), Poly(L-Milchsäure) (PLLA), Poly(L-Milchsäure-co-Glykolsäure) (PLLGA), Poly(L-Milchsäure-co-Glykolid-co-Caprolacton) (PLLA-GA-CL), und Kombinationen davon ist.

13. Die Vorrichtung des Anspruchs 11, wobei das amorphe, oder im Wesentlichen amorphe Polymer der Beschichtung Poly(D,L-Milchsäure-co-Glykolsäure) (PLGA) ist.

14. Die Vorrichtung des Anspruchs 13, wobei das Poly(D,L-Milchsäure-co-Glykolsäure) (PLGA) ausgewählt aus der Gruppe bestehend aus Poly(D,L-Milchsäure-co-Glykolsäure) (PLGA) 50/50, Poly(D,L-Milchsäure-co-Glykolsäure) (PLGA) 75/25, Poly(D,L-Milchsäure-co-Glykolsäure) (PLGA) 90/10, und Kombinationen davon ist.

15. Die Vorrichtung des Anspruchs 11, wobei das amorphe, oder im Wesentlichen amorphe Polymer der Beschichtung ein Poly(Lactid-Glycolid-Caprolacton)Terpolymer ist.

16. Die Vorrichtung des Anspruchs 11, wobei die Beschichtung 1 Gew.-% bis 35 Gew.-% Polymerkristallinität umfasst.

17. Die Vorrichtung des Anspruchs 11, wobei die Beschichtung 2 Gew.-% bis 30 Gew.-% Polymerkristallinität umfasst.

18. Die Vorrichtung des Anspruchs 11, wobei der dynamichen Verlustmodul der Beschichtung gemessen in einem linearen viskoelastischen Bereich bei einer Schwingungsfrequenz von 1 Radiant/Sekunde 2 X 10⁴ oder geringer ist.

19. Eine implantierbare medizinische Vorrichtung umfassend eine Beschichtung:
die Beschichtung umfassend:
eine Polymermischungszusammensetzung, wobei die Polymermischungszusammensetzung folgendes umfasst:
ein teilkristallines Polymer mit einem Gewichtsmittel des Molekulargewichts, das von 75.000 bis 300.000 reicht;
ein amorphes, oder im Wesentlichen amorphes Polymer mit einem Gewichtsmittel des Molekulargewichts, das von 75.000 bis 300.000 reicht;
wobei
das teilkristalline Polymer zwischen 2 Gew.-% und 75 Gew.% der Summe des teilkristallinen und des amorphen oder im Wesentlichen amorphen Polymers entspricht;
das teilkristalline Polymer ausgewählt aus der Gruppe bestehend aus Poly(D-Milchsäure) (PDLA), Poly(L-Milchsäure) (PLLA), Poly(L-Milchsäure-co-Glykolsäure) (PLLGA), Poly(L-Milchsäure-co-Glykolid-co-Caprolacton) (PLLA-GA-CL), und Kombinationen davon ist,
und das amorphe, oder im Wesentlichen amorphe Polymer ausgewählt aus der Gruppe bestehend aus Poly(D,L-Lactid-Glycolid), Poly(Lactid-Glycolid-Caprolacton)Terpolymeren, und Kombinationen davon ist,
wobei
die effektive Glasübergangstemperatur der Beschichtung -60 °C oder höher ist; die Beschichtung 0,5 Gew.-% bis 50 Gew.-% Polymerkristallinität umfasst;
die Beschichtung ein Wassergehalt von 10% oder weniger nach der Sterilisation umfasst; und
die effektive Glasübergangstemperatur die gemessene Glasübergangstemperatur vor der Exposition gegenüber Ethylenoxid-Sterilisation ist und, wenn mehr als eine Glasübergangstemperatur für die Mischung beobachtet wird, dann ist die effektive Glasübergangstemperatur die geringste der zwei oder mehreren deutlich beobachtbaren Glasübergangstemperaturwerten.

## Revendications

1. Une couche qui comprend:
une composition de mélange de polymères, comprenant ladite composition de mélange de polymères:
un polymère semi-cristallin ayant une moyenne en poids du poids moléculaire allant de 75.000 à 300.000;
un polymère amorphe, ou essentiellement amorphe, ayant une moyenne en poids du poids moléculaire allant de 75.000 à 300.000, et dans laquelle le polymère amorphe, ou essentiellement amorphe, est choisi dans le groupe constitué du poly(acide D,L-lactique-co-acide glycolique) (PLGA), polyéthylène glycol-bloc-poly(acide D,L-lactique) (PEG-PLA), polyéthylène glycol-bloc-poly(acide D,L-lactique-co-acide glicolique) (PEG-PLGA), terpolymères de poly(lactide-glycolide-caprolactone), et combinaisons de ceux-ci.
dans laquelle
le polymère semi-cristallin est entre 2% et 75% en poids de la somme du polymère semi-cristallin et du polymère amorphe, ou essentiellement amorphe;
dans laquelle
la température de transition vitreuse effective de la couche es - 60 °C ou supérieure;
la transition de fusion d'une région du polymère cristallin, ou au moins une transition de fusion d'une région du polymère cristallin, s'il y a plus d'une transition de fusion du polymère, est 70 °C ou supérieure;
la couche comprend de 0,5% à 50% en poids de cristallinité de polymère; et la température de transition vitreuse effective est la température de transition vitreuse mesurée avant l'exposition à la stérilisation avec de l'oxyde d'éthylène et, si plus d'une température de transition vitreuse est observée pour le mélange, alors la température de transition vitreuse effective est la valeur la plus basse des deux ou plusieurs valeurs de température de transition vitreuse distinctement observables.

2. La couche de la revendication 1, dans laquelle le polymère semi-cristallin est choisi dans le groupe constitué du poly(acide D-lactique) (PDLA), poly(acide L-lactique) (PLLA), poly(acide L-lactique-co-acide glycolique) (PLLGA), poly(acide L-lactique-co-glycolide-co-caprolactone) (PLLA-GA-CL), et des combinaisons de ceux-ci.

3. La couche de la revendication 1, dans laquelle le polymère amorphe, ou essentiellement amorphe, est le poly(acide D,L-lactique-co-acide glycolique (PLGA).

4. La couche de la revendication 3, dans laquelle le poly(acide D,L-lactique-co-acide glycolique) (PLGA) est choisi du groupe constitué du poly(acide D,L-lactique-co-acide glycolique) (PLGA) 50/50, poly(acide D,L-lactique-co-acide glycolique) (PLGA) 75/25, poly(acide D,L-lactique-co-acide glycolique) (PLGA) 90/10, et des combinaisons de ceux-ci.

5. La couche de la revendication 1, dans laquelle le polymère amorphe, ou essentiellement amorphe, est le terpolymère poly(lactide-glycolide-caprolactone).

6. La couche de la revendication 1, dans laquelle la couche comprend de 1% à 35% en poids de cristallinité de polymère.

7. La couche de la revendication 1, dans laquelle la couche comprend de 2% à 30% en poids de cristallinité de polymère.

8. La couche de la revendication 1, dans laquelle le module de perte de cisaillement dynamique, lorsqu'il est mesuré dans l'intervalle viscoélastique linéaire à une fréquence d'oscillation de 1 radian/seconde, est 2 X 10⁴ ou inférieur.

9. La couche de la revendication 1, comprenant la couche en outre un médicament.

10. La couche de la revendication 9, étant le médicament choisi dans le groupe constitué d'évérolimus, le zotarolimus, la déxaméthasone, et des combinaisons de ceux-ci.

11. Un dispositif médical implantable comprenant la couche selon la revendication 1.

12. Le dispositif de la revendication 11, dans lequel le polymère semi-cristallin de la couche est choisi dans le groupe constitué du poly(acide D-lactique) (PDLA), poly(acide L-lactique) (PLLA), poly(acide L-lactique-co-acide glycolique) (PLLGA), poly(acide L-lactique-co-glycolide-co-caprolactone) (PLLA-GA-CL), et des combinaisons de ceux-ci.

13. Le dispositif de la revendication 11, dans lequel le polymère amorphe, ou essentiellement amorphe de la couche est le poly(acide D,L-lactique-co-acide glycolique) (PLGA).

14. Le dispositif de la revendication 13, dans lequel le poly(D,L-acide lactique-co-acide glycolique) (PLGA) est choisi du groupe constitué du poly(acide D,L-lactique-co-acide glycolique) (PLGA) 50/50, poly(acide D,L-lactique-co-acide glycolique) (PLGA) 75/25, poly(acide D,L-lactique-co-acide glycolique) (PLGA) 90/10, et des combinaisons de ceux-ci.

15. Le dispositif de la revendication 11, dans lequel le polymère amorphe, ou essentiellement amorphe de la couche est le terpolymère poly(lactide-glycolide-caprolactone).

16. Le dispositif de la revendication 11, dans lequel la couche comprend de 1% à 35% en poids de cristallinité de polymère.

17. Le dispositif de la revendication 11, dans lequel la couche comprend de 2% à 30% en poids de cristallinité de polymère.

18. Le dispositif de la revendication 11, dans lequel le module de perte de cisaillement dynamique de la couche, lorsqu'il est mesuré dans l'intervalle viscoélastique linéaire à une fréquence d'oscillation de 1 radian/seconde, est 2 X 10⁴ ou inférieur.

19. Un dispositif médical implantable comprenant une couche:
comprenant la couche:
une composition de mélange de polymères, comprenant ladite composition de mélange de polymères:
un polymère semi-cristallin ayant une moyenne en poids du poids moléculaire allant de 75.000 à 300.000;
un polymère amorphe, ou essentiellement amorphe, ayant une moyenne en poids du poids moléculaire allant de 75.000 à 300.000;
dans laquelle
le polymère semi-cristalline est entre 2% et 75% en poids de la somme du polymère semi-cristalline et du polymère amorphe, ou essentiellement amorphe;
le polymère semi-cristallin est choisi dans le groupe constitué du poly(àcide D-lactique) (PDLA), poly(acide L-lactique) (PLLA), poly(acide L-lactique-co-acide glycolique) (PLLGA), poly(acide L-lactique-co-glycolide-co-caprolactone) (PLLA-GA-CL), et des combinaisons de ceux-ci.
et le polymère amorphe, ou essentiellement amorphe, est choisi du groupe constitué du poly(D,L-lactide-glycolide), du terpolymère poly(lactide-glycolide-caprolactone), et des combinaisons de ceux-ci,
dans laquelle
la température de transition vitreuse effective de la couche est -60 °C
ou supérieure; la couche comprend de 0,5% à 50% en poids de cristallinité de polymère;
la couche a une teneur en eau de 10% ou moins après la stérilisation; et la température de transition vitreuse effective est la température de transition vitreuse mesurée avant l'exposition à la stérilisation avec de l'oxyde d'éthylène et, si plus d'une température de transition vitreuse est observée pour le mélange, alors la température de transition vitreuse effective es la valeur la plus basse des deux ou plusieurs valeurs de température de transition vitreuse distinctement observables.
